# EUROPÄISCHE PATENTSCHRIFT

(11) **EP 1 379 622 B1**
(45) Veröffentlichungstag und Bekanntmachung des Hinweises auf die Patenterteilung: **12.11.2008**
(21) Anmeldenummer: 02740476.3
(22) Anmeldetag: 17.04.2002
(51) Int. Cl.: C12M 1/20

(54) **VERFAHREN UND VORRICHTUNG ZUM KULTIVIEREN UND/ODER VERTEILEN VON PARTIKELN**
METHOD AND DEVICE FOR CULTIVATING AND/OR DISTRIBUTING PARTICLES
PROCEDE ET DISPOSITIF DE CULTURE ET/OU DE REPARTITION DE PARTICULES

(30) Priorität: 18.04.2001 DE 10118905
(43) Veröffentlichungstag der Anmeldung: 14.01.2004
(73) Patentinhaber: Evotec AG, 22525 Hamburg (DE)
(72) Erfinder: MICHAELSEN, Nico, 22305 Hamburg (DE); SOLLBÖHMER, Olaf, 21224 Rosengarten/Eckel (DE)
(74) Vertreter: von Kirschbaum, Alexander
(86) Internationale Anmeldenummer: PCT/EP2002/004238
(87) Internationale Veröffentlichungsnummer: WO 2002/099033

(56) Entgegenhaltungen:
- WO-A-02/24861
- DE-U- 9 209 540
- DE-U- 29 815 276
- US-A- 5 817 510

## Beschreibung

Die Erfindung betrifft ein Verfahren und eine Vorrichtung zum Kultivieren von Partikeln, insbesondere von Zellen und Zelllinien. Ferner betrifft die Erfindung ein Verfahren zum Verteilen von Partikeln in Vertiefungen eines Probenträgers.

Zur Untersuchung von kultivierbaren Partikeln, wie Zellen und Zelllinien ist es erforderlich, zuvor entsprechende Partikel- bzw. Zellkulturen anzulegen. Derartige Zellkulturen können sodann beispielsweise durch Zugabe biologischer und/oder chemischer Substanzen auf Ihre Reaktionsfähigkeit untersucht werden. Hiermit kann beispielsweise die Wirkung von biologisch aktiven und/ oder pharmazeutisch wirksamen Verbindungen überprüft werden. Hierzu ist eine Vielzahl von Tests erforderlich. Um diese in möglichst kurzer Zeit und wirtschaftlich durchführen zu können, bestehen insbesondere Bestrebungen die zu untersuchende Probenmenge zu verringern. Dies ist erforderlich, da die Kosten für die Herstellung der Substanzen sowie für deren fachgerechte Entsorgung äußerst hoch sind.

Zur Verringerung der Probenvolumina ist es bekannt, z.B. adhärente Zellen auf geeigneten Probenträgern mit einer Vielzahl von Vertiefungen, beispielsweise auf Mikrotiterplatten mit 1536 oder 2080 Vertiefungen (Wells) einzusäen. Ferner kann es sich bei Probenträgern um Chips handeln, die als Vertiefungen beispielsweise Reservoirs und Kanäle aufweisen, wobei unterschiedliche Reservoirs oder Kanäle miteinander verbunden sein können. Bei der Untersuchung mit Hilfe von derartigen Chipstrukturen werden Probenflüssigkeiten beispielsweise zusätzlichen elektrischen Feldern o.dgl. ausgesetzt.

Die mit adhärenten Zellen versehenen Probenträger werden anschließend vorzugsweise in einen Inkubator, beispielsweise bei 37°C und einer kohlendioxidhaltigen Atmosphäre von beispielsweise 5 - 7 % Kohlendioxid inkubiert. Hierbei findet eine Adaption der Zellen gegenüber der in dem Inkubator herrschenden Umgebungsbedingungen statt, so dass die Zellen an der Oberfläche des Probenträgers adhärent werden. Untersuchungen haben ergeben, dass nach einer Inkubationszeit von 12 - 24 Stunden ein Großteil der Zellen adhärent ist. Da die Kultivierung der Zellen innerhalb der Vertiefungen mit kleinen Volumina, insbesondere weniger als 10 µl je Vertiefung durchgeführt wird, wurde bereits bei einer Inkubationszeit von 12 - 24 Stunden eine starke Abnahme von Nährstoffen, wie beispielsweise Vitaminen und Proteinen festgestellt. Durch diese Nährstoffabnahme wird die Vitalität der Zellen stark beeinträchtigt. Längere Kultivierungszeiten sind daher nicht möglich. Insbesondere bei empfindlichen Zellen ist eine Inkubation in derart kleinen Gefäßen überhaupt nicht möglich.

Wenn nach der Inkubationszeit die eingesäten, d. h. die mit beispielsweise adhärenten Zellen versehenen Platten dem Untersuchungsprozess, wie einem Hochdurchsatz-Screening-Prozess zugeführt werden, besteht ferner das Problem, dass sich in den Vertiefungen noch proteinhaltige Nährstoffe befinden. Diese können mit den Assayreagenzen, die zur Untersuchung den einzelnen Vertiefungen des Probenträgers zugeführt werden, Reaktionen hervorrufen, durch die die Messergebnisse verfälscht werden.

Eine Verfälschung der Messergebnisse tritt häufig auch auf Grund der Tatsache auf, dass in den einzelnen Vertiefungen des Probenträgers unterschiedliche Anzahlen Partikel vorhanden sind.

Aufgabe der Erfindung ist es, ein Verfahren und eine Vorrichtung zur Kultivierung und/oder Verteilung von Partikeln, insbesondere Zellen zu schaffen, mit dem eine Verbesserung der Messergebnisse erzielt werden kann.

Die Lösung der Aufgabe erfolgt erfindungsgemäß durch ein Verfahren gemäß Anspruch 1 bzw. 3 sowie eine Vorrichtung gemäß Anspruch 22.

Im ersten Schritt werden in Vertiefungen eines Probenträgers kultivierbare Partikel, insbesondere Zellen oder Zelllinien, eingebracht. Bei kultivierbaren Partikeln handelt es sich um Partikel, wie Zellen, die einen Stoffwechsel durchführen. Das Einbringen bzw. Einsäen der Partikel erfolgt beispielsweise durch geeignete Pipettier- oder Dispensiervorrichtungen. Bei dem Probenträger handelt es sich vorzugsweise um eine Titerplatte mit beispielsweise 1536 oder 2080 Wells, wobei jedes Well vorzugsweise ein Volumen von weniger als 10 µl, insbesondere von weniger als 5 µl aufweist. Ferner kann es sich bei Probenträgern um Chips mit als Vertiefungen ausgebildeten Kanälen und/oder Reservoirs handeln.

Anschließend werden die Vertiefungen mit einem gemeinsamen Deckel verschlossen. Der Deckel verschließt stets mehrere Vertiefungen. Vorzugsweise werden sämtliche Vertiefungen eines Probenträgers mit einem einzigen Deckel verschlossen. Der Deckel und/oder der Probenträger sind derart ausgebildet, dass zwischen einer Deckelunterseite und den Vertiefungen ein Zwischenraum besteht. Hierzu ist beispielsweise der Deckel mit einem Rand versehen, so dass die Deckelinnenseite einen Abstand zu dem Probenträger aufweist. Ebenso kann der Probenträger eine die Vertiefungen rahmenförmig umgebende Erhöhung aufweisen, mit der der Deckel verbunden wird, so dass wiederum ein Abstand zwischen der Deckelinnenseite und den Vertiefungen ausgebildet ist. Ferner weist der Deckel und/oder der Probenträger mindestens eine mit dem Zwischenraum in Verbindung stehende Öffnung auf.

Durch diese mindestens eine Öffnung wird erfindungsgemäß der Zwischenraum mit einem Partikelkulturmedium gefüllt. Zumindest ein Teil des Partikelkulturmediums wird während eines Kultivierungszeitraums ausgetauscht. Wenn beispielsweise nur eine Öffnung vorgesehen ist, erfolgt der Austausch des Kulturmediums auf die Weise, dass zumindest ein Teil des Kulturmediums entnommen und anschließend der Zwischenraum wieder mit frischem Kulturmedium befüllt wird.

Vorzugsweise wird der Probenträger vor dem Einbringen der kultivierbaren Partikel sterilisiert. Die Sterilisation kann beispielsweise durch eine Sterilisationsflüssigkeit und/oder Hitze erfolgen.

Mittels erfindungsgemäßen Verfahren ist es möglich, auf wirtschaftliche Weise zellbasierte Assays für das Hochdurchsatz-Screening herzustellen. Insbesondere ist mit dem erfindungsgemäßen Verfahren eine Kultivierung über mehrere Tage möglich, da den Zellen kontinuierlich oder in Intervallen, frisches Medium zugeführt werden kann. Durch diesen Mediumaustausch kann auf eine CO₂-Versorgung verzichtet wird, da der pH-Wert nicht in den zelltoxischen Bereich gelangt. Besonders geeignet ist das erfindungsgemäße Verfahren für zelluläre Translokationsereignisse, wie z. B. Membran zu Zytoplasmatranslokationen oder Zytoplasma zu Nukleus Translokationen. Ferner ist das Verfahren insbesondere geeignet für funktionelle Rezeptorbindungsassays, Second-Messenger-Assays, Reporterassays, Zytotoxizitätsassays, Zellzyklusassays, Proliferationsassays, Assays zu morphologischen Zellveränderungen, Protein-Protein-Interaktionsassays, Zell-Zell-Aggregationsassays etc.

Vorzugsweise wird der Probenträger mit einer Benetzungsflüssigkeit benetzt. Dies erfolgt vorzugsweise vor dem Sterilisieren des Probenträgers. Zur Benetzung des Probenträgers kann eine geeignete Benetzungseinrichtung verwendet werden, die eine blasenfreie Befüllung ermöglicht.

Um das Auftreten von Luftblasen zu vermeiden, durch die die Zellkultivierung gestört werden könnte, erfolgt die Befüllung des Zwischenraums vorzugsweise vollständig. Vorzugsweise sind mindestens zwei Öffnungen vorgesehen, so dass auch ein kontinuierlicher Austausch des Partikelkulturmediums möglich ist. Hierbei wird durch eine Öffnung Partikelkulturmedium zugeführt und durch die andere abgeführt. Dies kann auch diskontinuierlich erfolgen.

Durch das erfindungsgemäße Vorsehen eines Zwischenraums, der kontinuierlich oder diskontinuierlich mit Partikelkulturmedium gefüllt werden kann, so dass zumindest ein Teil des Partikelkulturmediums während des Kultivierungszeitraums austauschbar ist, können erheblich längere Kultivierungszeiträume realisiert werden. Es ist möglich, Kultivierungszeiträume zu realisieren, die insbesondere länger als 24, insbesondere auch länger als 72 Stunden betragen. Es ist somit möglich, z. B. auch langsam wachsende Zellen oder Zelllinien zu kultivieren. Ein besonderer Vorteil des erfindungsgemäßen Verfahrens liegt darin, dass keine zusätzliche Gasversorgung, insbesondere eine Kohlendioxidversorgung, erforderlich ist. Es ist daher nicht erforderlich mittels aufwendiger Prozesse und Vorrichtungen sicherzustellen, dass die Zellen oder andere kultivierbare Partikel stets mit der richtigen Menge an Gasen, insbesondere Kohlendioxid, versorgt werden. Die Versorgung mit Kohlendioxid dient hierbei zur pH-Regulierung in dem Partikelkulturmedium. Dies ist insbesondere deswegen aufwendig, weil sowohl eine Unterversorgung als auch eine Überversorgung mit Kohlendioxid, d.h. eine pH-Wert-Änderung, schädlich für die Zellen ist. Da auch die übrigen Nährstoffe und dergleichen unmittelbar durch das Partikelkulturmedium, insbesondere das Zellkulturmedium den Partikeln zugeführt werden können, müssen keine besonderen Umgebungsbedingungen in einem Inkubator oder dergleichen eingehalten werden. Bei der Kultivierung von Partikeln muss die Umgebung äußerst steril sein. Da es sich bei der erfindungsgemäßen Vorrichtung um einen Probenträger mit einem den Kultivierungsraum verschließenden Deckel handelt, kann diese Vorrichtung in einer Umgebung eingesetzt werden, die nur erheblich geringeren Sterilitätsanforderungen genügen muss. Ferner ist die ansonsten in Inkubatoren auftretende Verdunstung des Partikelkulturmediums durch das erfindungsgemäße Vorsehen eines Deckels vermieden.

Vorzugsweise werden die Vertiefungen des Probenträgers bereits vor dem Einbringen von kultivierbaren Partikeln verschlossen. Die kultivierbaren Partikel können nunmehr durch Zuführen einer Partikelsuspension über die mit dem Zwischenraum verbundene Öffnung in den Zwischenraum eingebracht werden. Hierbei wird der Zwischenraum vorzugsweise vollständig mit Partikelsuspension befüllt. Gegebenenfalls durch Inkubation des mit dem Deckel verschlossenen Probenträgers erfolgt eine Adhäsion der Partikel, insbesondere der Zellen an der Oberfläche des Probenträgers. Hierbei ist vorzugsweise die Oberfläche derart ausgewählt, dass eine bevorzugte Adhäsion der Partikel in den Vertiefungen erfolgt. Dies kann beispielsweise dadurch erreicht werden, dass eine die Vertiefungen verschließende Bodenplatte aus einem die Adhäsion begünstigenden Material hergestellt ist. Vorzugsweise ist die Innenseite des Deckels sowie übrige Bereiche, in denen eine Adhäsion der Partikel nicht erwünscht ist, aus einem Material, an dem die Partikel nicht anhaften. Ebenso ist eine geeignete Beschichtung dieser Bereiche möglich.

Das Verschließen der Vertiefungen des Probenträgers vor dem Einbringen der kultivierbaren Partikel hat den Vorteil, dass das Einbringen einer Partikelsuspension in den Zwischenraum erheblich einfacher ist als das genaue Dosieren und Platzieren der Partikel in den Vertiefungen. Dies ist insbesondere dann der Fall, wenn Probenträger mit besonders kleinen Vertiefungen, die beispielsweise ein Volumen von weniger als 10 µl, insbesondere weniger als 5 µl, aufweisen, verwendet werden.

Vorzugsweise wird der Deckel nicht vor dem Einbringen von kultivierbaren Partikeln, sondern bereits vor dem Sterilisieren des Probenträgers mit dem Probenträger verbunden. Zur Sterilisierung des Probenträgers kann somit über die mindestens eine mit dem Zwischenraum verbundene Öffnung eine Sterilisationsflüssigkeit dem Zwischenraum zugeführt werden. Dies hat den Vorteil, dass der Probenträger und seine Vertiefungen zusammen mit der Innenseite des Deckels in einem Arbeitsgang sterilisiert werden. Da der Deckel dicht mit dem Probenträger abschließt, ist der Sterilisierungsvorgang erheblich einfacher, da die Umgebung, in der die Sterilisierung stattfindet nicht dieselben hohen Sterilitätsanforderungen erfüllen muss, wie die Vertiefungen und die Innenseite des Deckels. Als Sterilisationsflüssigkeit kann beispielsweise 70%ige Ethanollösung verwendet werden.

Insbesondere, wenn der Zwischenraum mindestens zwei Öffnungen aufweist, von denen eine als Einlass- und eine als Auslassöffnung dient, ist es möglich, die Sterilisationsflüssigkeit aus dem Zwischenraum zu entfernen, indem Partikelsuspension durch die Einlassöffnung zugeführt wird und dabei die Sterilisationsflüssigkeit durch die Auslassöffnung herausgedrückt wird. Um die Sterilisationsflüssigkeit vollständig aus dem Zwischenraum zu entfernen, geht jedoch eine gewisse Menge an Partikelsuspension verloren, da im Grenzbereich zwischen der Sterilisationsflüssigkeit und der Partikelsuspension ein Vermischen der beiden Flüssigkeiten stattfindet, so dass auch ein Teil der Partikelsuspension aus dem Zwischenraum entfernt werden muss, um sicherzustellen, dass die Sterilisationsflüssigkeit vollständig aus dem Zwischenraum herausgedrückt wurde.

Es ist daher vorteilhaft, die Sterilisationsflüssigkeit vor dem Einbringen von Partikelsuspensionen zu entfernen. Dies kann beispielsweise durch Entleeren des Zwischenraums über die mindestens eine mit dem Zwischenraum verbundene Öffnung erfolgen. Vorzugsweise erfolgt das Entfernen der Sterilisationsflüssigkeit jedoch durch Zuführen einer Prozessflüssigkeit, die die Sterilisationsflüssigkeit verdrängt. Hierbei wird wiederum die Prozessflüssigkeit durch eine von mindestens zwei mit dem Zwischenraum verbundenen Öffnungen zugeführt und die Sterilisationsflüssigkeit aus der anderen Öffnung herausgedrückt. Das Verwenden einer Prozessflüssigkeit ist erheblich kostengünstiger als das Verwenden der Partikelsuspension zum Herausdrücken der Sterilisationsflüssigkeit. Als Prozessflüssigkeit wird eine Flüssigkeit verwendet, bei der ein Vermischen mit der nachträglich zugeführten Partikelsuspension für diese nicht schädlich ist. Beispielsweise kann als Prozessflüssigkeit die Trägerflüssigkeit der Partikelsuspension verwendet werden, so dass die Prozessflüssigkeit der Partikelsuspension mit der Ausnahme entspricht, dass sie keine kultivierbaren Partikel enthält.

Der erfindungsgemäße Austausch von Kultivierungsmedium während eines Kultivierungszeitraums, durch den erheblich längere Kultivierungszeiten realisiert werden können, erfolgt vorzugsweise kontinuierlich. Dies hat den Vorteil, dass über den gesamten Kultivierungszeitraum im Wesentlichen dieselbe Zusammensetzung des Partikelkulturmediums gewährleistet ist. Ferner hat das kontinuierliche Austauschen des Kultivierungsmediums den Vorteil, dass beim Austausch verschiedene Behandlungslösungen ö.ä. zusammen mit dem Kultivierungsmedium dem Zwischenraum zugeführt werden können.

Ferner können unterschiedliche Kultivierungsmedien nacheinander zugeführt werden. Vorzugsweise wird vor dem Zuführen eines neuen Kultivierungsmediums der Zwischenraum zwischen Deckel und Probenträger beispielsweise mit Prozeßflüssigkeit gespült.

Bei der Partikelsuspension kann es sich auch um eine Suspension handeln, die sedimentierende Partikel enthält. Derartige Partikel haften nicht oder nur zu einem sehr geringen Teil an den Wänden der Vertiefungen des Probenträgers. Eine sedimentierende Partikel aufweisende Partikelsuspension kann ebenfalls, wie vorstehend beschrieben, vorzugsweise durch Zuführen der Partikelsuspension in den Zwischenraum, den einzelnen Vertiefungen zugeführt werden.

Da eine sedimentierende Partikel aufweisende Partikelsuspension beim Befüllen des Zwischenraums mit Partikelkulturmedium von diesem ausgewaschen würde, wird vorzugsweise vor dem Befüllen des Zwischenraums mit Partikelkulturmedium eine die Partikel in den Vertiefungen zurückhaltende Membran oder ein Gitter vorgesehen. Hierzu kann beispielsweise der die Vertiefungen verschließende Deckel abgenommen, z.B. eine Membran oder ein Gitter über die Vertiefungen, d. h. auf den Probenträger gelegt und anschließend der Deckel wieder mit dem Probenträger verbunden werden. Vorzugsweise werden jedoch zwei unterschiedliche Deckel verwendet, so dass der erste die Vertiefungen verschließende Deckel abgenommen wird und ein zweiter eine entsprechende Membran oder ein Gitter aufweisender Deckel mit dem Probenträger verbunden wird. Hierdurch entfällt der äußerst komplizierte Arbeitsschritt des Einlegens einer Membran oder eines Gitters über die Vertiefungen.

Bei einer bevorzugten Ausführungsform wird vor dem Befüllen des Zwischenraums mit Partikelkulturmedium die Trägerplatte zusammen mit dem die Vertiefungen verschließenden Deckel derart angeordnet, dass in dem Zwischenraum vorhandene Luft durch eine Auslassöffnung austreten kann. Ein derartiges Anordnen der Trägerplatte kann auch bereits früher, beispielsweise bereits vor dem Einbringen der die kultivierbaren Partikel aufweisenden Partikelsuspension oder auch schon vor dem Sterilisieren durch Zuführen von Sterilisationsflüssigkeit in den Zwischenraum geschehen. Vorzugsweise wird als Luftauslassöffnung eine der mindestens zwei bei dieser Ausführungsform mit dem Zwischenraum verbundenen Öffnungen verwendet. Diese Öffnung ist sodann am höchsten Punkt des Zwischenraums vorgesehen.

Es ist auch möglich, eine gesonderte Auslassöffnung, die ausschließlich zum Auslass von Luft dient, vorzusehen. Eine derartige Öffnung kann beispielsweise mit einem hydrophoben Filter verschlossen sein, so dass Flüssigkeit durch diese Öffnung nicht entweichen kann. Diese Öffnung kann beispielsweise bei einer horizontal angeordneten Titerplatte im Deckel vorgesehen sein. Hierzu ist die Deckelinnenseite derart ausgebildet, dass Luftblasen an der Innenseite des Deckels in Richtung der Auslassöffnung aufsteigen. Beispielsweise ist die Innenseite des Deckels pyramidenförmig, wobei an der Pyramidenspitze die Auslassöffnung angeordnet ist.

Bei einer bevorzugten Ausführungsform des erfindungsgemäßen Verfahrens wird die Trägerplatte vor dem Zuführen der Trägerplatte zu einem Untersuchungsprozess, wie beispielsweise einem Screening-Prozess gewaschen. Hierdurch erfolgt ein Entfernen überflüssigen Partikelkulturmediums, das die Untersuchungsergebnisse beeinträchtigen könnte. So werden die Untersuchungsergebnisse beispielsweise durch noch vorhandene Proteine verfälscht. Das Waschen erfolgt vorzugsweise mit einer Pufferflüssigkeit. Entsprechend dem Zuführen von Flüssigkeiten zu dem Zwischenraum über eine Einlassöffnung erfolgt auch das Zuführen der Waschflüssigkeit, vorzugsweise über diese eine bzw. mehrere Einlassöffnungen.

Vor dem Zuführen des Probenträgers zu einem Untersuchungsprozess wird vorzugsweise ein Teil der Waschflüssigkeit entfernt. Dies kann beispielsweise durch Absaugen der Waschflüssigkeit durch eine mit dem Zwischenraum verbundene Öffnung erfolgen. Anschließend wird der Deckel unter sterilen Bedingungen entfernt.

Zur Verbesserung der Untersuchungsergebnisse in dem Untersuchungsprozess wird anschließend mit Hilfe einer Entnahmevorrichtung aus jeder der Vertiefungen soviel Waschflüssigkeit entnommen, dass die Vertiefungen mit annähernd demselben Volumen befüllt sind bzw. dasselbe Volumenniveau haben. Zur Abführung einer überschüssigen Menge an Waschflüssigkeit je Vertiefung können beispielsweise Kapillaren bis auf das gewünschte Niveau in die Vertiefungen abgesenkt werden. Durch eine an die Kapillaren angeschlossene Saugeinrichtung oder durch auftretende Kapillarkräfte wird die überschüssige Flüssigkeit abgesaugt. Ebenso können beispielsweise bei Chips Abführkanale o.dgl. vorgesehen sein. Als Entnahmevorrichtung kann ferner eine Fritte, vorzugsweise aus Keramik verwendet werden. Diese weist zu den Vertiefungen komplementäre Ansätze auf, so dass beim Aufsätzen der Fritte auf den Probenträger sämtliche Ansätze gleich tief in die einzelnen Vertiefungen hineinragen und somit bis zu diesem Niveau Flüssigkeit aus den Vertiefungen aufnehmen.

Das vorstehend beschriebene erfindungsgemäße Verfahren zum Kultivieren von Partikeln, insbesondere von Zellen und Zelllinien ist insbesondere für den Einsatz im Hochdurchsatz-Screening geeignet.

Die Erfindung betrifft ferner ein Verfahren zum Verteilen von Partikeln in einem Probenträger. Durch das erfindungsgemäße Verfahren ist es möglich, Partikel in einer Vielzahl von Vertiefungen eines Probenträgers, wie beispielsweise einer Titerplatte, homogen zu verteilen. Bei den Partikeln kann es sich beispielsweise auch um Beads (synthetische polymere Partikel) handeln.

Erfindungsgemäß wird hierzu ein Probenträger mit einer Vielzahl von Vertiefungen vorzugsweise sterilisiert. Anschließend werden die Vertiefungen des Probenträgers mit einem gemeinsamen Deckel verschlossen. Hierbei ist der Deckel und/oder der Probenträger derart ausgebildet, dass zwischen einer Deckelinnenseite und den Vertiefungen ein Zwischenraum ausgebildet ist. Ferner weist der Deckel oder Probenträger mindestens eine mit dem Zwischenraum in Verbindung stehende Öffnung auf. Im nächsten Schritt wird der Zwischenraum mit einer Partikelflüssigkeit befüllt. Bei der Partikelflüssigkeit handelt es sich um eine die in die Vertiefungen einzubringenden Partikel aufweisende Flüssigkeit. Die Partikelflüssigkeit wird während eines Partikel-Verteilzeitraums zumindest teilweise ausgetauscht. Hierdurch entsteht eine Bewegung der Partikelflüssigkeit innerhalb des Zwischenraums. Untersuchungen haben ergeben, dass überraschenderweise eine ausreichende homogene Verteilung der Partikel über die einzelnen Vertiefungen erfolgt.

Vorstehendes Verfahren zur Verteilung von Partikeln in Probenträgern hat den Vorteil, dass es nicht mehr erforderlich ist, jeder Vertiefung einzeln über ein Pipettier- oder Dispensierverfahren eine bestimmte Menge an Partikelflüssigkeit zuzuführen. Eine solche Zuführung ist mit herkömmlichen Dispensier- oder Pipettierverfahren für Volumina im Micro- oder Nanoliterbereich besonders aufwendig oder nicht durchführbar. Insbesondere weist das erfindungsgemäße Verfahren auch gegenüber Pipettier- oder Dispensierverfahren bei einem erheblich geringeren Aufwand vergleichbare Homogenität der Partikelanzahl je Vertiefung bei deutlich geringerem Aufwand auf. Werden als Probenträger beispielsweise Chips eingesetzt, so können die Vertiefungen unterschiedlich große Volumina aufweisen. Die Anzahl der Partikel ist dann auch von den Volumina abhängig. Bezogen auf die Volumeneinheit ist jedoch weiterhin eine hohe Homogenität der Partikelverteilung mit Hilfe des erfindungsgemäßen Verfahrens je Volumeneinheit erzielbar.

Vorzugsweise wird der Probenträger mit den entsprechend homogen verteilten Partikeln anschließend einem Untersuchungsprozess zugeführt. Da es sich bei den Partikeln auch um Zellen oder andere kultivierbare Partikel handeln kann, kann nach dem Verteilen der Partikel auf die einzelnen Vertiefungen auch ein Kultivierungsprozess durchgeführt werden. Bei dem Kultivierungsprozess kann es sich um das vorstehend beschriebene erfindungsgemäße Verfahren zum Kultivieren von Partikeln handeln.

Das erfindungsgemäße Verfahren zum Verteilen von Partikeln kann ferner, wie vorstehend anhand des Verfahrens zum Kultivieren von Partikeln beschrieben vorteilhaft weitergebildet sein.

Die Partikel können in den zwischen der Deckelinnenseite und den Vertiefungen vorhandenen Zwischenraum unmittelbar durch die Partikelflüssigkeit zugeführt werden. Es ist beispielsweise aber auch möglich, die Partikel erst in dem Zwischenraum zu erzeugen. Beispielsweise kann die Partikelflüssigkeit entsprechenden Umgebungsbedingungen, wie Wärme, magnetischen/elektrischen Kräften u. Ä., ausgesetzt werden, so dass sich auf Grund der Umgebungsbedingungen Partikel bilden. Ebenso kann die Partikelflüssigkeit, die in den Zwischenraum eingeleitet wird, Partikel aufweisen, die beispielsweise durch Ultraschall oder ein Zentrifugieren aufgespalten werden, so dass sich die Partikelanzahl erhöht.

Bei den beiden vorstehend beschriebenen Verfahren ist es insbesondere möglich, das in den Zwischenraum einströmende und/oder ausströmende Medium zu messen und zu untersuchen. Hierdurch können Vergleiche zwischen dem ein-und ausströmenden Medium durchgeführt werden, aus denen sich beispielsweise Rückschlüsse auf den Stoffwechsel der Zellen ziehen lassen.
Ebenso ist es möglich, den mit dem Deckel verschlossenen Probenträger z. B. in einem Inkubator oder Ofen zu temperieren. Hierbei kann beispielsweise zusätzlich die dem Zwischenraum zugeführte Flüssigkeit vorgeheizt werden. Insbesondere ist es möglich, den Probenträger auf eine Metallplatte zum Erwärmen der im Zwischenraum befindlichen Flüssigkeit anzuordnen. Über die Metallplatte ist es auch möglich, einen Temperaturgradienten in die in dem Zwischenraum vorhandene Flüssigkeit einzuprägen.

Über die mit dem Zwischenraum verbundene Öffnung bzw. die Öffnungen können zusätzlich zu den Partikeln Viren und/oder Bakterien eingebracht werden, um deren Reaktion mit den in den Vertiefungen vorhandenen Partikeln, insbesondere Zellen, zu untersuchen. Ebenso können nacheinander unterschiedliche Flüssigkeiten dem Zwischenraum zugeführt werden. Um die in den einzelnen Vertiefungen vorgesehenen Partikel beispielsweise zu einem späteren Zeitpunkt untersuchen zu können, ist es auch möglich, die mit Partikeln, insbesondere Zellen, bestückten Probenträger einzufrieren und zur Untersuchung zu einem späteren Zeitpunkt wieder aufzutauen.

Die Erfindung betrifft ferner eine Vorrichtung zum Kultivieren und/oder Verteilen von Partikeln, insbesondere von Zellen und Zelllinien. Die erfindungsgemäße Vorrichtung ist zur Durchführung der beiden vorstehend beschriebenen erfindungsgemäßen Verfahren besonders geeignet. Hierzu weist die Vorrichtung einen Probenträger mit einer Vielzahl von Vertiefungen auf. Die Vertiefungen sind mit einem Deckel verschlossen, wobei der Deckel derart ausgebildet ist, dass er mehrere, vorzugsweise sämtliche Vertiefungen verschließt. Ferner ist der Deckel und/oder der Probenträger derart ausgebildet, dass zwischen einer Deckelinnenseite und den Vertiefungen ein Zwischenraum ausgebildet ist. Hierzu weist beispielsweise der Deckel einen in Richtung des Probenträgers weisenden umlaufenden Rand auf. Ebenso kann der Probenträger einen die Vertiefungen umgebenden Rahmen aufweisen, auf den der Deckel aufgesetzt wird. Zum Einbringen der vorstehend beschriebenen Prozessflüssigkeiten in den Zwischenraum, weist der Deckel und/oder der Probenträger mindestens eine mit dem Zwischenraum in Verbindung stehende Öffnung auf. Mit Hilfe der erfindungsgemäßen Vorrichtung kann das vorstehend beschriebene Verfahren auf einfache Weise durchgeführt werden. Vorzugsweise weist der Deckel und/oder der Probenträger mindestens zwei Öffnungen auf, wobei eine Öffnung als Einlassöffnung und eine als Auslassöffnung dient.

Um das Zu- und Abführen von Prozessflüssigkeiten zu erleichtern, sind mindestens zwei Öffnungen, vorzugsweise einander gegenüberliegend angeordnet. Dies ist insbesondere vorteilhaft, wenn eine nachfolgende Prozessflüssigkeit eine in dem Zwischenraum befindliche Prozessflüssigkeit aus dem Zwischenraum herausdrücken soll.

Bei einer weiteren bevorzugten Ausführungsform der erfindungsgemäßen Vorrichtung ist mindestens eine Einlass- und eine Auslassöffnung an derselben Seite des Deckels und/oder Probenträgers vorgesehen. Dies hat den Vorteil, dass bei schräg oder senkrecht gestellter Vorrichtung die Ein- und Auslassöffnung gut zugänglich sind und automatisch durch die, vorzugsweise oben angebrachte Auslassöffnung Luft entweichen kann.

Der Probenträger weist vorzugsweise eine Deckelaufnahme auf, so dass die Lage des Deckels auf dem Probenträger definiert ist. Hierzu kann der Probenträger beispielsweise einen umlaufenden Rahmen aufweisen, innerhalb dem der Deckel eingesetzt wird. Der umlaufende Rahmen dient zusätzlich zum Abdichten des Zwischenraums. Hierzu weit der Deckel vorzugsweise an der Berührungsfläche zwischen Deckel und Probenträger eine Dichtung auf.

Um den Deckel am Probenträger zu halten, kann der Deckel und/oder der Probenträger beispielsweise eine Rastverbindung aufweisen, so dass der Deckel beim Aufsetzen auf den Probenträger einrastet. Ferner ist es möglich, durch eine entsprechende Anordnung von Magneten und/oder Metallelementen ein magnetisches Halten des Deckels zu realisieren. Es ist ferner möglich, den Deckel über geeignete Klammern oder Schnappverschlüsse mit dem Probenträger zu verbinden.

Vorzugsweise weist der Deckel und/oder der Probenträger zusätzlich eine Entlüftungsöffnung auf, die mit einem hydrophoben Filter versehen sein kann.

Wenn mit der erfindungsgemäßen Vorrichtung Partikelsuspensionen untersucht werden sollen, deren Partikel sedimentieren und daher nicht oder nur geringfügig an den Wänden der Vertiefungen des Probenträgers haften, weist vorzugsweise der Deckel eine Membran oder ein Gitter auf, die derart an dem Deckel vorgesehen ist, dass die Membran oder das Gitter die Vertiefungen gegenüber dem Zwischenraum abschließt. Hierdurch ist sichergestellt, dass beispielsweise beim kontinuierlichen Austauschen von Partikelkulturmedium die in den Vertiefungen vorhandenen Partikel nicht ausgespült werden.

Um z. B. die Lage der Membran festzulegen und über die Vertiefungen zu spannen, weist der Probenträger zur Deckelaufnahme vorzugsweise eine Ausnehmung auf, in die ein Rand des Deckels eingesetzt wird. Hierbei ist die Ausnehmung vorzugsweise tiefer als die Oberseite des Probenträgers, an denen die Öffnungen der Vertiefungen angeordnet sind. Durch Einsetzen des Deckels in den Probenträger wird z. B. die von dem Deckel gehaltene Membran somit automatisch über die Vertiefungen gespannt, so dass die Membran die Vertiefungen gegenüber dem Zwischenraum automatisch abtrennt.

Vorzugsweise ist der Deckel und/oder der Boden transparent. Es ist hierdurch möglich, auf einfache Weise in der erfindungsgemäßen Vorrichtung ablaufende Prozesse, beispielsweise mit Hilfe einer Kamera, zu beobachten. Ferner können durch einen für entsprechende Strahlung durchlässigen Deckel und/oder Boden in der Vorrichtung ablaufende Prozesse beeinflusst oder initiiert werden. Diese Ausführungsform ist insbesondere bei Chip-Strukturen vorteilhaft, bei denen z. B. die Substanzen dielektrischen Feldkräften ausgesetzt sind oder mit optischen Pipetten gehandhabt werden.

Da der Probenträger insbesondere zum Anwenden in modernen Hochdurchsatz-Screening-Anlagen ausgebildet ist, weisen die Vertiefungen ein Volumen von weniger als 10 µl, insbesondere weniger als 5 µl auf. Ferner sind die Vertiefungen vorzugsweise derart angeordnet, dass sie standardisierten Titerplatten entsprechen. Es sind somit in einem Probenträger beispielsweise 1536 oder 2080 Vertiefungen vorgesehen.

Bei dem Probenträger kann es sich ferner um eine Chip-Struktur handeln, die als Vertiefungen, beispielsweise Kanäle und/oder Reservoirs aufweist. Ebenso kann als Probenträger auch ein planarer Probenträger verwendet werden. Dieser weist beispielsweise chemisch und/oder mechanisch behandelte Bereiche auf, an denen Substanzen vorzugsweise gut bzw. schlecht haften. Es kann sich hierbei um hydrophobe und hydrophile Bereiche handeln. Diese Bereiche entsprechen sodann Vertiefungen der vorstehend anhand der Verfahren bzw. der Vorrichtung beschriebenen Probenträgern. Es ist ferner möglich, im Deckel Sensoren zur Überwachung bzw. Überprüfung der Kultivierungsbedingungen, wie beispielsweise dem pH-Wert, der Temperatur etc., vorzusehen.

Nachfolgend wird die Erfindung anhand bevorzugter Ausführungsformen, unter Bezugnahme auf die anliegenden Zeichnungen näher erläutert.
Es zeigen:
- Fig. 1: eine schematische Schnittansicht einer ersten Ausführungsform,
- Fig. 2: eine schematische Ansicht der ersten Ausführungsform von oben in Richtung des Pfeils II in Fig. 1,
- Fig. 3: eine schematische Draufsicht einer zweiten Ausführungsform,
- Fig. 4: eine schematische Schnittansicht einer dritten Ausführungsform, und
- Fig. 5: eine schematische perspektivische Ansicht einer stapelförmigen Anordnung von erfindungsgemäßen Probenträgern mit Deckel.

Die erfindungsgemäße Vorrichtung weist beispielsweise einen Probenträger 10 mit einer Vielzahl von Vertiefungen 12 auf. Die Vertiefungen 12 sind als beispielsweise durchgehende zylindrische Öffnungen in einem Basisteil 14 vorgesehen. Das Basisteil 14 ist mit einem Bodenteil 16, bei dem es sich beispielsweise um eine Glasplatte handelt, verbunden. Durch das Bodenteil 16 werden die Vertiefungen 12 nach unten verschlossen. Ferner weist der Probenträger 10 einen das Basisteil 14 umgebenden Rahmen 18 auf. Der Rahmen 18 weist eine Dicke auf, die größer ist als die Dicke des Bodenteils 16, zusammen mit dem Basisteil 14. Hierdurch ist ein Absatz 20 zwischen dem Rahmen 18 und dem Basisteil 14 ausgebildet. In dem dargestellten Ausführungsbeispiel ist der Absatz 20 rechteckig.

Der Absatz 20 dient zur Aufnahme eines Deckels 22, der sämtliche Vertiefungen 12 verschließt. Der Deckel 22 weist einen in Richtung des Basisteils 14 weisenden Rand 24 auf. Der Rand 24 ist ebenfalls rechteckig, wobei die Außenabmessungen des Randes 24 den Innenabmessungen des Absatzes 20 entsprechen. Die Lage des Deckels 22 relativ zu dem Probenträger 10 ist somit durch den Absatz 20 festgelegt.

Aufgrund des in Richtung des Basisteils 14 weisenden Randes 24 des Deckels 22 ist zwischen den Vertiefungen 12 und einer Innenseite 26 des Deckels 22 ein Zwischenraum 28 ausgebildet.

In dem Rand 24 ist ein Kanal 30 vorgesehen, der eine mit dem Zwischenraum 28 in Verbindung stehende Öffnung 32 aufweist. Das der Öffnung 32 gegenüberliegende Ende des Kanals ist mit einem rohrförmigen Einlassstutzen 34 verbunden. Durch den Einlassstutzen 34 kann Prozessflüssigkeit in den Zwischenraum 28 geleitet werden. Auf der gegenüberliegenden Seite ist in dem Rand 24 ein zweiter Kanal 36 vorgesehen, der ebenfalls eine mit dem Zwischenraum 28 in Verbindung stehende Öffnung 38 aufweist. Der Kanal ist ferner mit einem Auslassstutzen 40 verbunden, durch den Prozessflüssigkeit aus dem Zwischenraum 28 abgeleitet werden kann.

Der Deckel 22 kann von dem Probenträger 10 abgenommen und auf diesen aufgesetzt werden. Um einen dichten Zwischenraum 28 zu schaffen, weist der Deckel an einer an dem Basisteil 14 anliegenden Unterseite 42 eine Dichtung 44 auf. Die Dichtung 44 ist im gesamten Rand 24 angeordnet, so dass es sich ebenfalls um eine rechteckige Dichtung handelt.

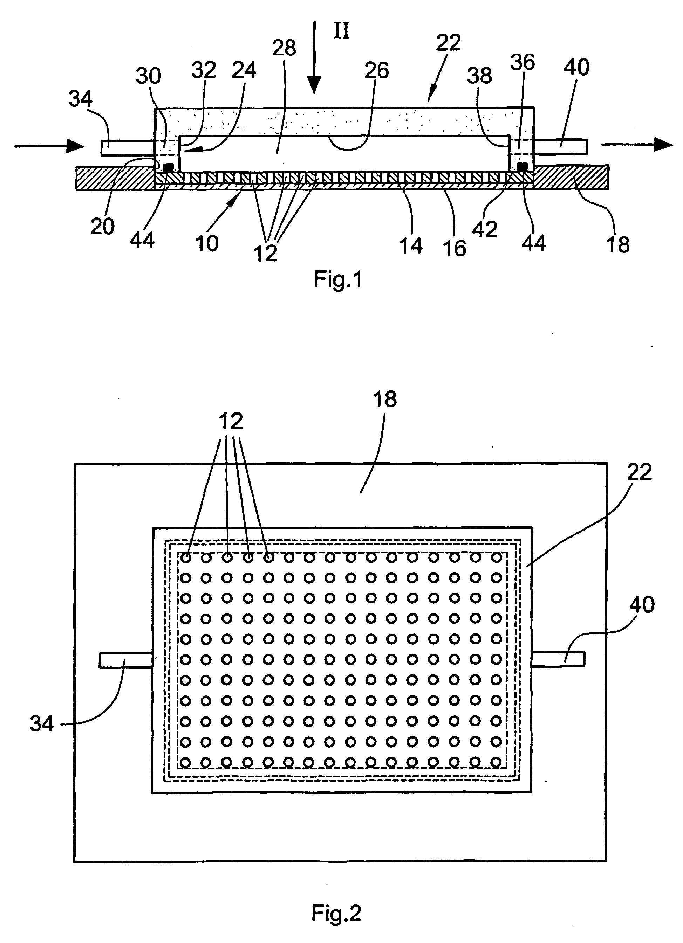

Ferner weist die in den Fign. 1 und 2 dargestellte erste Ausführungsform der erfindungsgemäßen Vorrichtung ein Verschlusselement auf, durch das der Deckel 22 an dem Probenträger 10 fixiert werden kann.

Wie aus Fig. 2 ersichtlich ist, ist der Deckel 22 aus transparenten Material, vorzugsweise aus transparentem Kunststoff. Dies hat den Vorteil, dass der innerhalb des Zwischenraums 28 stattfindende Prozess von außen beobachtet, beeinflusst und kontrolliert werden kann.

Der Deckel 22 kann ferner so ausgebildet sein, dass er auf dem Rahmen 18 aufliegt. Hierbei ist die Dichtung 44 vorzugsweise derart ausgebildet, dass weiterhin eine Abdichtung gegenüber der Oberseite des Basisteils 14 erfolgt. Die Dichtung ist hierbei vorzugsweise Z-förmig ausgebildet, so dass ein Teil der Dichtung auf der Oberseite des Rahmens 18 unter dem Deckel 22 angeordnet ist, die Dichtung sodann in Fig. 1 nach unten in Richtung des Basisteils 14 verläuft und das zweite Ende der Dichtung auf der Oberseite des Basisteils 14 anliegt. Zur Verringerung des Todvolumens kann an der Deckelinnenseite 26 ein Zusatzlayer vorgesehen sein. Vorzugsweise ist die Z-förmige ausgebildete Dichtung sowie der Zusatzlayer derart ausgebildet, dass das Zu- bzw. Ableiten von Flüssigkeiten durch den Einlass- bzw. Auslassstutzen 34,40 nicht beeinträchtigt ist.

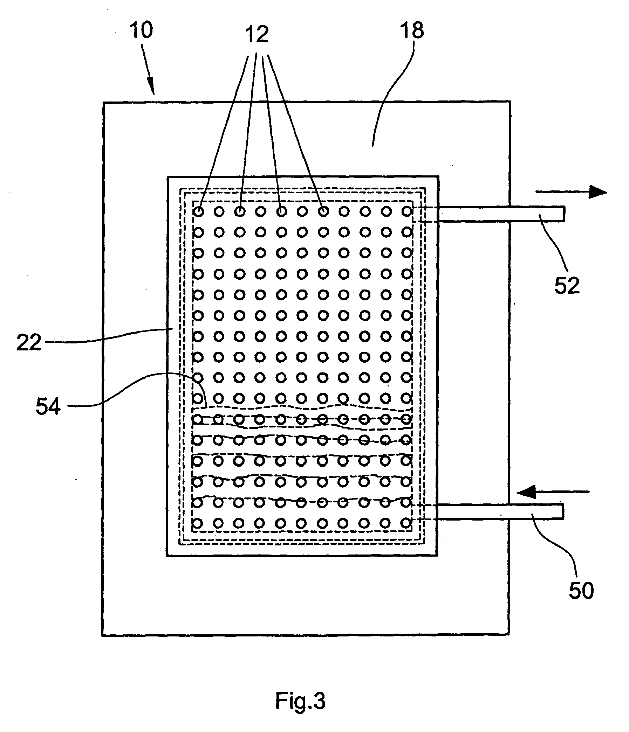

Bei der in Fig. 3 dargestellten Ausführungsform handelt es sich um eine der in den Fig. 1 und 2 dargestellten Ausführungsform ähnlichen Ausführungsform. Gleiche Bestandteile sind daher mit gleichen Bezugszeichen bezeichnet.

Der in der zweiten Ausführungsform dargestellte Probenträger 10 ist vollständig mit dem der ersten Ausführungsform identisch. Der einzige Unterschied zu der ersten Ausführungsform besteht in der Anordnung des Einlass- und Auslassstutzens. Bei der zweiten Ausführungsform weisen ein Einlassstutzen 50 sowie ein Auslassstutzen 40 in dieselbe Richtung. Sie sind an derselben Seite des Deckels 22 vorgesehen und ebenfalls mit einem Zwischenraum verbunden.

Bei dieser Ausführungsform ist es auf einfache Weise möglich, die Vorrichtung, d. h. den mit dem Deckel 22 verschlossenen Probenträger 10 schräg oder senkrecht anzuordnen. Es ist möglich, durch den unten liegenden Einlassstutzen 50 eine Prozessflüssigkeit 54 in den Zwischenraum einzuleiten, deren Niveau sodann langsam in dem Zwischenraum steigt. Hierbei wird automatisch die in dem Zwischenraum befindliche Luft durch den Auslassstutzen 40 aus dem Zwischenraum verdrängt. Bei langsamen Einleiten der Prozessflüssigkeit 54 in den Zwischenraum verbleiben in dem Zwischenraum keine Blasen. Ferner ist es durch diese Anordnung der erfindungsgemäßen Vorrichtung möglich, durch eine über den Einlassstutzen 50 in den Zwischenraum eingeführte Prozessflüssigkeit eine bereits in dem Zwischenraum vorhandene Prozessflüssigkeit durch den Auslassstutzen 40 aus dem Zwischenraum zu verdrängen.

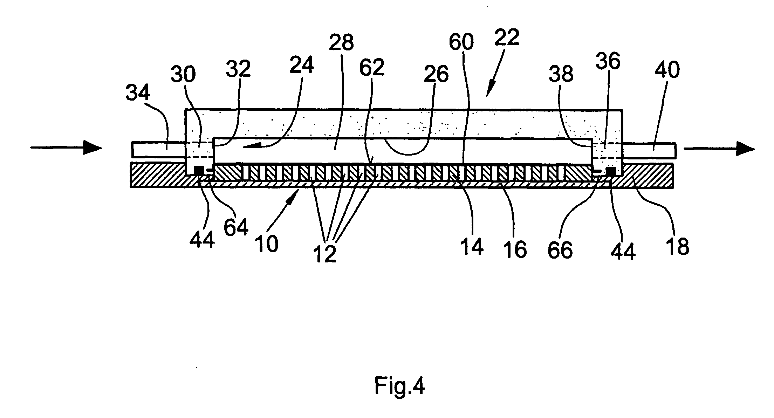

Bei der dritten Ausführungsform (Fig. 4) handelt es sich um eine Ausführungsform, die besonders zur Durchführung eines Kultivierungsverfahrens geeignet ist, bei welchem die Partikel sedimentieren und nicht in den Vertiefungen 12 haften. Gleiche Bestandteile sind wiederum mit gleichen Bezugszeichen bezeichnet.

Der wesentliche Unterschied zu den vorstehend beschriebenen Ausführungsformen besteht darin, dass der Deckel 22 zusätzlich eine Membran 60 aufweist. Die Membran 60 liegt auf einer Oberseite 62 des Basisteils 14, im dem die Vertiefungen 12 angeordnet sind auf. Die Membran 60 ist umlaufend in dem Rand 24 des Deckels 22 verankert. Um ein sicheres Verschließen der Vertiefungen 12 mit der Membran 60 zu gewährleisten, weist der Probenträger 10 eine umlaufende Ausnehmung 64 auf. Die Ausnehmung 64 ist rahmenförmig und dient zur Aufnahme des Randes 24 des Deckels 22. Die Ausnehmung 64 ist etwas tiefer als die Oberseite 62 des Basisteils 14. Da die Verankerung 66 der Membran 60 in dem Rand 24 bei geschlossenem Deckel 22 unterhalb der Oberseite 62 des Basisteils 14 liegt, wird die Membran 60 automatisch beim Aufsetzen des Deckels 22 auf den Probenträger 10 über die Vertiefungen 12 gespannt.

Ferner kann der Deckel mit einem Barcode oder einer ähnlichen Kennung versehen sein. Mit Hilfe des Barcodes kann eine Steuerung der gesamten Anlage erfolgen. Beispielsweise kann die Anlage aus dem Barcode entnehmen, welche Art von Kultivierungs- und/oder Untersuchungsverfahren durchgeführt werden soll.

Vorteilhaft ist ferner eine Anordnung mehrerer Probenträger mit Deckel in Reihe, so dass Partikelkulturmedium nacheinander durch mehrere Zwischenräume 28 hintereinandergeschalteter Vorrichtungen strömt. Dies hat den Vorteil, dass auf sehr effektive Weise gleichzeitig mehrere Probenträger mit denselben Partikeln, beispielsweise Zellen, bestückt werden können.

Es ist ferner möglich, mehrere Probenträger parallel oder seriell anzuordnen und mit Partikelkulturmedium oder einer anderen Flüssigkeit zu befüllen.

Um Partikel in einem Probenträger in den einzelnen Vertiefungen 12 anzuordnen, deren spezifisches Gewicht leichter als das der Flüssigkeit ist, kann die erfindungsgemäße Vorrichtung derart angeordnet werden, dass der Deckel 22 nach unten weist. Die in den Zwischenraum 28 eingebrachten Partikel steigen sodann innerhalb des Zwischenraums 28 nach oben und verteilen sich wie vorstehend beschrieben auf die einzelnen Vertiefungen 12, da sie auf Grund ihrer Dichte nach oben steigen.

Als Bodenteil 16 wird insbesondere ein transparenter, vorzugsweise aus Glas bestehender, Boden verwendet. Es ist somit möglich, durch den Boden hindurch Untersuchungen und Beobachtungen mit Hilfe von optischen Messmethoden u. dgl. durchzuführen. Ebenso ist der Deckel 22 vorzugsweise transparent, so dass das in dem Zwischenraum 28 stattfindende Verfahren auf einfache Weise beobachtet werden kann.

Ebenso ist es möglich, durch einen transparenten Deckel 22 sowie durch einen transparenten Boden 16 die in dem Zwischenraum 28 befindlichen Partikel zu manipulieren. Dies kann beispielsweise durch ein Einstrahlen von Energie, Licht oder durch Handhaben mittels einer optischen Pinzette erfolgen.

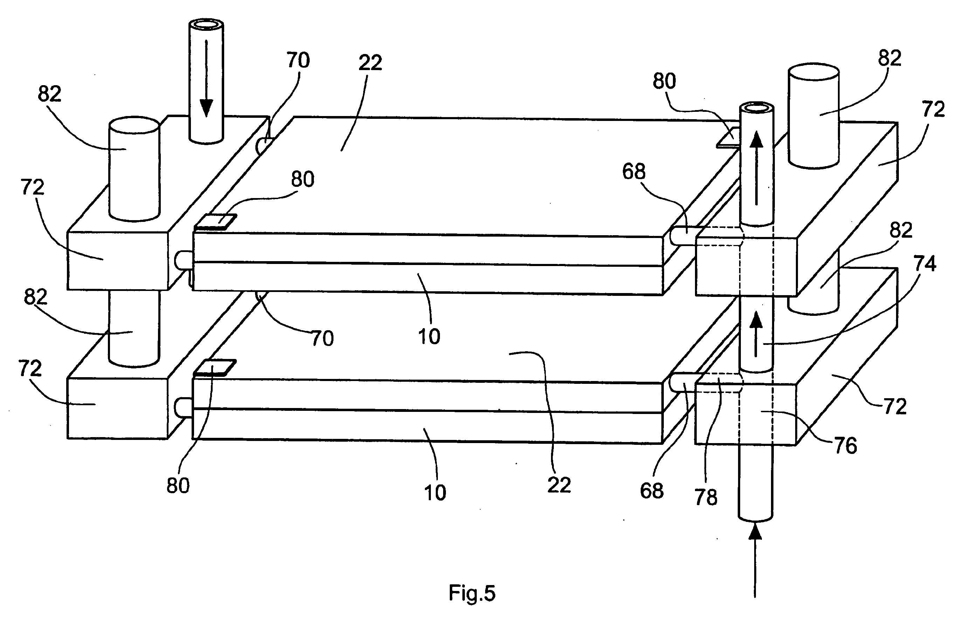

Zur gleichzeitigen Befüllung mehrerer Probenträger 10 können diese stapelförmig angeordnet sein (Fig. 5). Bei den mit einem Deckel 22 verschlossenen Probenträgern kann es sich um die anhand der Fign. 1 - 4 beschriebenen Ausführungsformen der Erfindung handeln. Der zwischen dem Deckel 22 und dem Probenträger 10 vorgesehene Zwischenraum 28 ist entsprechend den vorstehend beschriebenen Ausführungsformen jeweils mit einem Einlassstutzen 68 und einem Auslassstutzen 70 verbunden, durch die Flüssigkeit dem Zwischenraum zu- bzw. abgeführt werden kann. Entsprechend der dargestellten Ausführungsform kann eine Vielzahl derartiger Probenträger 10 mit Deckeln 22 stapelförmig übereinander angeordnet werden. Hierzu sind Halterungen 72 vorgesehen. Jeweils zwei einander gegenüber angeordnete Halterungen 72, die identisch ausgebildet sind, nehmen eine Trägerplatte 10 zusammen mit einem Deckel 22 auf. Hierzu weist jede Halterung 72 eine mit einem rohrförmigen Kanal 74 oder dgl. versehenen Durchgangskanal 76 auf. Innerhalb der Halterung 72 ist der Durchgangskanal 76 zu einem Kanal 78 verzweigt. In eine mit dem Kanal 78 verbundene Ausnehmung wird der Einlassstutzen 68 bzw. der Auslassstutzen 70 eingesteckt, so dass eine Fluidverbindung zwischen dem Kanal 76 und dem Einlass- bzw. Auslassstutzen 68, 70 besteht.

Ferner weist jede Halterung 72 eine Klemmvorrichtung 80 auf, die den Deckel 22 zusammen mit dem Probenträger 10 U-Förmig umgibt und somit den Probenträger 10 fest geschlossen hält. Ferner weist die Halterung 72 Verbindungselemente 82, die zum verbinden zweier übereinander angeordneter Halterungen 72 dienen auf. Die Verbindungselemente 82 und die Halterung 72 sind derart ausgebildet, dass mehrere Halterungen 72 übereinander angeordnet und miteinander fixiert werden können.

Bei der in Fig. 5 dargestellten stapelförmigen Anordnung wird somit Fluid über den Kanal 76 und den von diesem abzweigenden Kanal 78 in den Einlassstutzen 68 geleitet. Durch diesen gelangt die Flüssigkeit in den Zwischenraum 28 und wird durch den Auslassstutzen 70, der diagonal gegenüber dem Einlassstutzen 68 angeordnet ist, wieder abgeführt. Ein Teil der Flüssigkeit fließt durch den Kanal 76 und das Rohr 74 in Richtung einer über der ersten Halterung 72 angeordneten zweiten Halterung 72. In dieser wird wiederum durch die Abzweigung des Kanals 76 ein Teil der Flüssigkeit in den Zwischenraum 28 geleitet. Zwei übereinander angeordnete Ebenen eines Probenträgers 10 mit Deckel 22 zusammen mit den beiden Halterungen 72 sind identisch ausgebildet.

Aufgrund der in Fig. 5 dargestellten Ausgestaltung der Halterungen 72 werden die übereinander angeordneten Probenträger 10 parallel mit Flüssigkeit befüllt bzw. entleert. Es ist ebenso möglich, die Kanalführung in den Halterungen derart vorzusehen, dass die Flüssigkeit schlangenlinienförmig geführt ist und somit die Probenträger 10 nacheinander von einer Flüssigkeit durchströmt werden. Je nach Ausgestaltung der Kanäle an den Halterungen 72 ist somit eine parallele sowie eine serielle Befüllung der Probenträger 10 möglich.

## Patentansprüche

1. Verfahren zum Kultivieren von Partikeln, insbesondere Zellen, mit den Schritten:
Einbringen von kultivierbaren Partikeln, insbesondere Zellen, in Vertiefungen (12) eines eine Vielzahl von Vertiefungen (12) aufweisenden Probenträgers (10),
Verschließen der Vertiefungen (12) mit einem gemeinsamen Deckel (22), wobei der Deckel (22) und/oder der Probenträger (10) derart ausgebildet sind, dass zwischen einer Deckelinnenseite (26) und den Vertiefungen (12) ein Zwischenraum (28) ausgebildet ist, und der Deckel (22) und/oder der Probenträger (10) mindestens eine mit dem Zwischenraum (28) in Verbindung stehende Öffnung (32, 38) aufweist,
Befüllen des Zwischenraums (28) mit einem Partikelkulturmedium,
Austausch zumindest eines Teils des Partikelkulturmediums während eines Kultivierungszeitraums,
und Zuführen des Probenträgers (10) zu einem Untersuchungsprozess.

2. Verfahren nach Anspruch 1, bei welchem die Vertiefungen vor dem Einbringen von kultivierbaren Partikeln mit dem Deckel (22) verschlossen werden und das Eindringen der Partikel durch Zuführen einer Partikelsuspension in den Zwischenraum (28) erfolgt.

3. Verfahren nach einem der Ansprüche 1 oder 2, bei welchem der Probenträger 10 und/oder der Deckel 22 vor dem Einbringen der kultivierbaren Partikel bzw. der Partikelflüssigkeit sterilisiert wird.

4. Verfahren nach einem der Ansprüche 1 - 3, bei welchem der Probenträger vorzugsweise in einer Benetzungseinrichtung, insbesondere vor dem Sterilisieren, mit einer Benetzungsflüssigkeit benetzt wird.

5. Verfahren nach Anspruch 4, bei welchem die Vertiefungen (12) vor dem Sterilisieren mit dem Deckel (22) verschlossen werden und das Sterilisieren durch Zuführen einer Sterilisationsflüssigkeit in den Zwischenraum (28) erfolgt.

6. Verfahren nach Anspruch 5, bei welchem vor dem Einbringen der Partikel die Sterilisationsflüssigkeit entfernt wird.

7. Verfahren nach Anspruch 6, bei welchem das Entfernen der Sterilisationsflüssigkeit durch Zuführen einer Prozessflüssigkeit erfolgt, die die Sterilisationsflüssigkeit verdrängt.

8. Verfahren nach einem der Ansprüche 1 - 7, bei welchem der Austausch des Partikelkulturmediums kontinuierlich erfolgt.

9. Verfahren nach einem der Ansprüche 1 - 8, bei welchem nach dem Einbringen der Partikel der Probenträger (10) zur Adhäsion der Partikel in den Vertiefungen (12) einem Inkubator zugeführt wird.

10. Verfahren nach Anspruch 9, bei welchem das Material eines Bodenteils (16) der Vertiefungen (12) derart gewählt ist, dass die Partikel vorzugsweise am Boden der Vertiefungen (12) anhaften.

11. Verfahren nach einem der Ansprüche 1 - 8, bei welchem nach dem Einbringen der Partikel der Probenträger zur Sedimentation der Partikel in den Vertiefungen (12) einem Inkubator zugeführt wird.

12. Verfahren nach einem der Ansprüche 1 - 11, bei welchem zur Verbesserung der Homogenität der Partikelverteilung in dem Zwischenraum (28) ein, vorzugsweise sämtliche Vertiefungen (12) überspannendes Gitter oder eine Membran vorgesehen ist.

13. Verfahren nach einem der Ansprüche 1 - 12, bei welchem vor dem Befüllen mit Partikelkulturmedium eine die Partikel in den Vertiefungen (12) zurückhaltende Membran (60) vorgesehen wird.

14. Verfahren nach Anspruch 12 oder 13, bei welchem zum Vorsehen der Membran (60) oder des Gitters der die Vertiefungen (12) verschließende Deckel (22) abgenommen und ein die Membran (60) oder das Gitter tragender Deckel (22) aufgesetzt wird.

15. Verfahren nach einem der Ansprüche 1 - 14, bei welchem vor dem Befüllen des Zwischenraums (28) mit Partikelkulturmedium bzw. Partikelflüssigkeit der Probenträger (10) derart angeordnet wird, dass in dem Zwischenraum (28) vorhandene Luft durch eine Auslassöffnung (52) austreten kann.

16. Verfahren nach einem der Ansprüche 1 - 15, bei welchem der Probenträger vor dem Zuführen zu dem Untersuchungsprozess vorzugsweise mit einer Pufferflüssigkeit gewaschen wird.

17. Verfahren nach Anspruch 16, bei welchem nach dem Waschen der Deckel (22) vorzugsweise in steriler Umgebung abgenommen wird.

18. Verfahren nach Anspruch 17, bei welchem mit Hilfe einer Entnahmevorrichtung aus jeder Vertiefung (12) so viel Waschflüssigkeit entnommen wird, dass jede Vertiefung (12) dasselbe Flüssigkeitsniveau aufweist.

19. Verfahren nach einem der Ansprüche 1 - 18, bei welchem der Untersuchungsprozess ein Hochdurchsatz-Screening-Prozess ist.

20. Vorrichtung zur Durchführung des Verfahrens nach einem der Ansprüche 1 - 19, mit
einem eine Vielzahl an Vertiefungen (12) aufweisenden Probenträger (10),
einem Deckel (22) zum gemeinsamen Verschließen der Vertiefungen (12), wobei der Deckel (22) und/oder der Probenträger (10) derart ausgebildet sind, dass zwischen einer Deckelinnenseite (26) und den Vertiefungen (12) ein Zwischenraum (28) ausgebildet ist und
mindestens einer mit dem Zwischenraum (28) in Verbindung stehenden Öffnung (32,38).
**dadurch gekennzeichnet,**
**dass** der Deckel (22) und/oder der Probenträger (10) zusätzlich eine Entlüftungsöffnung aufweist, die vorzugsweise mit einem hydrophoben Filter versehen ist.

21. Vorrichtung nach Anspruch 20, **dadurch gekennzeichnet, dass** mindestens eine mit dem Zwischenraum (28) in Verbindung stehende Einlassöffnung (32) und mindestens eine Auslassöffnung (38) vorgesehen ist.

22. Vorrichtung nach Anspruch 21, **dadurch gekennzeichnet, dass** die Öffnungen (32,38) einander gegenüberliegen.

23. Vorrichtung nach Anspruch 21, **dadurch gekennzeichnet, dass** die Öffnungen (50,52) an einer Seite des Deckels (22) und/oder des Probenträgers (10) angeordnet sind.

24. Vorrichtung nach einem der Ansprüche 20 - 23, **dadurch gekennzeichnet, dass** der Deckel (22) einen umlaufenden Rand (24) aufweist.

25. Vorrichtung nach einem der Ansprüche 20 - 24, **dadurch gekennzeichnet, dass** der Probenträger (10) eine Deckelaufnahme (20) aufweist.

26. Vorrichtung nach einem der Ansprüche 20 - 25, **dadurch gekennzeichnet, dass** der Deckel (22) und/oder der Probenträger (10) eine den Zwischenraum (28) abdichtende Dichtung (44) aufweist.

27. Vorrichtung nach einem der Ansprüche 20 - 26, **dadurch gekennzeichnet, dass** der Deckel (22) eine Membran (60) oder ein Gitter aufweist, die derart angeordnet ist, dass die Membran (60) oder ein Gitter die Vertiefungen (12) gegenüber dem Zwischenraum (28) abtrennt.

28. Vorrichtung nach Anspruch 27, **dadurch gekennzeichnet, dass** der Probenträger zur Deckelaufnahme eine Ausnehmung (64) aufweist, die derart ausgebildet ist, dass die Membran (60) über die Vertiefungen (12) gespannt ist.

29. Vorrichtung nach einem der Ansprüche 20 -28, **dadurch gekennzeichnet, dass** das Volumen jeder Vertiefung (12) kleiner als 10 µl, insbesondere kleiner als 5 µl ist.

30. Vorrichtung nach einem der Ansprüche 20 -29, **dadurch gekennzeichnet, dass** ein transparenter Deckel (22) und/oder ein transparenter Boden (16) vorgesehen ist.

## Claims

1. Method for cultivating particles, in particular cells, comprising the steps of:
introducing cultivatable particles, in particular cells, into recesses (12) in sample carrier (10) comprising a plurality of recesses (12),
closing the recesses (12) with a common lid (22), the lid and/or the sample carrier (10) being configured such that a gap (28) is formed between an inner side (26) of the lid and the recesses (12) and the lid (22) and/or the sample carrier (10) has at least one opening (32, 38) in communication with the gap (28),
filling the gap (28) with a particle cultivation medium,
replacing at least a part of the particle cultivation medium during a cultivation period,
and transferring the sample carrier (10) to an analyzing process.

2. Method of claim 1, wherein the recesses are closed with the lid (22) prior to the introduction of cultivatable particles and the penetration of the particles is effected by introducing a particle suspension into the gap (28).

3. Method of one of claims 1 or 2, wherein the sample carrier (10) and/or the lid (22) is sterilized prior to the introduction of the cultivatable particles or the particle liquid.

4. Method of one of claims 1-3, wherein the sample carrier is preferably wetted in a wetting means with a wetting liquid, in particular before sterilizing.

5. Method of claim 4, wherein the recesses (12) are closed with the lid (22) prior to sterilizing and the sterilizing is effected by feeding a sterilizing liquid into the gap (28).

6. Method of claim 5, wherein the sterilizing liquid is removed prior to the introduction of the particles.

7. Method of claim 6, wherein the removal of the sterilizing liquid is effected by feeding a process liquid that displaces the sterilizing liquid.

8. Method of one of claims 1-7, wherein the replacement of the particle cultivation medium is continuous.

9. Method of one of claims 1-8, wherein, after the introduction of the particles, the sample carrier (10) is transferred to an incubator for the particles to adhere in the recesses (12).

10. Method of claim 9, wherein the material of a bottom part (16) of the recesses (12) is selected such that the particles preferably adhere to the bottom of the recesses (12).

11. Method of one of claims 1 - 8, wherein, after the introduction of the particles, the sample carrier is transferred to an incubator for the sedimentation of the particles in the recesses (12).

12. Method of one of claims 1 - 11, wherein, for an improvement of the homogeneity of the particle distribution, a mesh or a membrane is provided in the gap (28) that spans one, preferably all recesses (12).

13. Method of one of claims 1 - 12, wherein, prior to the filling with particle cultivation medium, a membrane 60) is provided that retains the particles in the recesses (12).

14. Method of claim 12 or 13, wherein to provide the membrane (60) or the mesh, the lid (22) closing the recesses (12) is removed and a lid (22) supporting the membrane (60) and or the mesh.

15. Method of one of claims 1 - 14, wherein, prior to the filling of the gap (28) with particle cultivation medium or particle liquid, the sample carrier (10) is arranged such that air present in the gap (28) can escape through an outlet opening (52).

16. Method of one of claims 1 - 15, wherein sample carrier is washed preferably with a buffer liquid prior to the transfer to the analyzing process.

17. Method of claim 16, wherein, after washing, the lid (22) is removed preferably in a sterile environment.

18. Method of claim 17, wherein, using a removal device, washing liquid is taken from each recess (12) such that each recess (12) contains the same level of liquid.

19. Method of one of claims 1 - 18, wherein the analyzing process is a high-throughput screening process.

20. Device for performing the method of one of claims 1-19, comprising
a sample carrier (10) provided with a plurality of recesses (12),
a lid (22) for closing the recesses (12) jointly, the lid (22) and/or the sample carrier (10) being configured such that a gap (28) is formed between an inner side (26) of the lid and the recesses (12), and
at least one opening (32, 38) in communication with the gap (28),
**characterized in that**
the lid (22) and/or the sample carrier (10) additionally comprises a venting opening preferably provided with a hydrophobic filter.

21. Device of claim 20, **characterized in that** at least one inlet opening (32) in communication with the gap (28) and at least one outlet opening (38) are provided.

22. Device of claim 21, **characterized in that** the openings (32, 38) are opposite each other.

23. Device of claim 21, **characterized in that** the openings (50, 52) are arranged on one side of the lid (22) and/or the sample carrier (10).

24. Device of one of claims 20-23, **characterized in that** the lid (22) has a circumferential edge (24).

25. Device of one of claims 20-24, **characterized in that** the sample carrier (10) comprises a seat (20) for the lid.

26. Device of one of claims 20-25, **characterized in that** the lid (22) and/or the sample carrier (10) comprises a seal (44) sealing the gap (28).

27. Device of one of claims 20-26, **characterized in that** the lid (22) comprises a membrane (60) or a mesh arranged such that the membrane (60) or mesh separates the recesses (12) from the gap (28).

28. Device of claim 27, **characterized in that** the sample carrier has a recess (64) for receiving the lid, which recess is formed such that the membrane (60) is spanned across the recesses (12).

29. Device of one of claims 20-28, **characterized in that** the volume of each recess (12) is less than 10 µl, in particular less than 5 µl.

30. Device of one of claims 20-29, **characterized in that** a transparent lid (22) and/ or a transparent bottom (16) is provided.

## Revendications

1. Procédé de culture de particules, à savoir de cellules, comprenant les étapes suivantes:
introduire des particules cultivables, à savoir des cellules, dans des concavités (12) d'un support échantillons (10) comportant plusieurs de concavités (12),
fermer les concavités (12) par un couvercle (22) commun, le couvercle (22) et/ou le support échantillons (10) sont agencés de façon qu'un espace intermédiaire (28) est formé entre une face intérieure (26) du couvercle et les concavités (12), et que le couvercle (22) et/ou le support échantillons (10) comprend au moins une ouverture (32, 38) en communication avec l'espace intermédiaire (28),
remplir l'espace intermédiaire (28) d'un fluide de culture de particules,
remplacer d'au moins une partie du fluide de culture de particules pendant le temps de culture,
et transférer le support échantillons (10) à un procès d'analyse.

2. Procédé selon la revendication 1, dans lequel les concavités sont fermées avec le couvercle (22) avant que les particules cultivables soient introduites, et la pénétration des particules est effectuée par conduisant une suspension de particules dans l'espace intermédiaire (28).

3. Procédé selon une des revendications 1 ou 2, dans lequel le support échantillons (10) et/ou le couvercle (22) est stérilisé avant d'introduire les particules cultivables ou le liquide de particules.

4. Procédé selon une queconque des revendications 1-3, dans lequel le support échantillons est mouillé d'un liquide de mouillage, de préférence dans un moyen de mouillage, en particulier avant d'être stérilisé.

5. Procédé selon la revendication 4, dans lequel les concavités (12) son fermées avec le couvercle (22) avant la stérilisation, et la stérilisation est effectuée par l'introduction d'un liquide de stérilisation dans l'espace intermédiaire (28).

6. Procédé selon la revendication 5, dans lequel le liquide de stérilisation est enlevé avant que les particules soient introduites.

7. Procédé selon la revendication 6, dans lequel l'enlèvement du liquide de stérilisation est effectué par l'introduction d'un liquide de procédé déplaçant le liquide de stérilisation.

8. Procédé selon une quelconque des revendications 1-7, dans lequel le remplacement du fluide de culture de particules est effectué en continu.

9. Procédé selon une quelconque des revendications 1-8, dans lequel, après l'introduction des particules, le support échantillons (10) est transféré à un incubateur pour l'adhésion des particules dans les concavités (12).

10. Procédé selon la revendication 9, dans lequel le matériau d'une partie de fond (16) des concavités (12) est choisi tel que les particules adhèrent de préférence au fond des concavités (12).

11. Procédé selon une quelconque des revendications 1-8, dans lequel après l'introduction des particules, le support échantillons est transféré à un incubateur pour la sédimentation des particules dans les concavités (12).

12. Procédé selon une quelconque des revendications 1 - 11, dans lequel pour l'amélioration de l'homogénéité de la répartition des particules dans l'espace intermédiaire (28) une grille ou une membrane recouvrant une, de préférence toutes les concavités (12).

13. Procédé selon une quelconque des revendications 1 - 12, dans lequel une membrane (60) est prévue retenant les particules dans les concavités (12), avant le remplissage de fluide de culture de particules.

14. Procédé selon la revendication 12 ou 13, dans lequel, pour installer la membrane (60) ou la grille, le couvercle (22) fermant les concavités (12) est enlevé et un couvercle (22) supportant la membrane (60) ou la grille est placé.

15. Procédé selon une quelconque des revendications 1 - 14, dans lequel, avant de remplir l'espace intermédiaire (28) de fluide de culture de particules ou de liquide de particules, le support échantillons (10) est arrangé de manière que de l'air présente dans l'espace intermédiaire (28) peut s'échapper d'une ouverture de sortie (52).

16. Procédé selon une quelconque des revendications 1 - 15, dans lequel le support échantillons est rincé de préférence avec un liquide tampon avant de transférer le support échantillons au procès d'analyse.

17. Procédé selon la revendication 16, dans lequel, après rinçage, le couvercle (22) est enlevé de préférence dans un milieu stérile.

18. Procédé selon la revendication 17, dans lequel on enlève, en utilisant un moyen d'enlèvement, autant de liquide de rinçage de chaque concavité (12) que chaque concavité (12) contient le même niveau de liquide.

19. Procédé selon une quelconque des revendications 1 - 18, dans lequel le procès d'analyse est un procès de criblage à haute capacité.

20. Dispositif pour l'exécution du procédé selon une quelconque des revendications 1-19, comprenant
un support échantillons (10) comprenant plusieurs concavités (12),
un couvercle (22) pour fermer les concavités (12) en commun, le couvercle (22) et/ou le support d'échantillons (10) étant agencés de manière qu'un espace intermédiaire (28) est formé entre une face intérieure (26) du couvercle et les concavités (12), et
au moins une ouverture (32, 38) en communication avec l'espace intermédiaire (28),
**caractérisé en ce que**
le couvercle (22) et/ou le support échantillons (10) comprend en outre une ouverture d'échappement d'air prévue, de préférence, d'un filtre hydrophobe.

21. Dispositif selon la revendication 20, **caractérisé en ce que** au moins une ouverture d'admission (32) en communication avec l'espace intermédiaire (28) et au moins une ouverture de sortie (38) sont prévues.

22. Dispositif selon la revendication 21, dans lequel les ouvertures (32, 38) s'opposent.

23. Dispositif selon la revendication 21, **caractérisé en ce que** les ouvertures (50, 52) sont disposées dans une coté du couvercle (22) et/ou du support échantillons (10).

24. Dispositif selon une quelconque des revendications 20-23, dans lequel le couvercle (22) comprend un bord (24) circulaire.

25. Dispositif selon une quelconque des revendications 20-24, dans lequel le support échantillons (10) comprend un logement (20) pour le couvercle.

26. Dispositif selon une quelconque des revendications 20-25, dans lequel le couvercle (22) et/ou le support échantillons (10) comprend un joint (44) pour étancher l'espace intermédiaire (28).

27. Dispositif selon une quelconque des revendications 20-26, dans lequel le couvercle (22) comprend une membrane (60) ou une grille agencée de manière que la membrane (60) ou la grille sépare les concavités (12) de l'espace intermédiaire (28).

28. Dispositif selon la revendication 27, dans lequel le support échantillons comprend un creux (64) pour recevoir le couvercle, le creux étant agencé de manière que la membrane (60) est tendue au-dessus des concavités (12).

29. Dispositif selon une quelconque des revendications 20-28, dans lequel la volume de chaque concavité (12) est moins que 10 µl, en particulier moins que 5 µl.

30. Dispositif selon une quelconque des revendications 20-29, dans lequel un couvercle (22) transparent et/ou an fond (16) transparent sont prévus.
